# EUROPEAN PATENT APPLICATION

(11) **EP 4 616 803 A1**
(43) Date of publication of application: **17.09.2025**
(21) Application number: 23888773.1
(22) Date of filing: 09.11.2023
(51) Int. Cl.: A61B 5/256, A61B 5/16, A61B 5/291, A61B 5/374, A61B 5/377

(54) **BIOSIGNAL MEASUREMENT UNIT AND CONDITION ANALYSIS SYSTEM**

(30) Priority: 11.11.2022 JP 2022181417
(71) Applicant: Keio University, Tokyo, 108-8345 (JP); LIFESCAPES Inc., Tokyo 106-0047 (JP)
(72) Inventor: USHIBA, Junichi, Yokohama-shi, Kanagawa 223-8522 (JP); FUKUDA, Mori, Yokohama-shi, Kanagawa 223-8522 (JP); MORIKAWA, Koji, Tokyo 106-0047 (JP)
(74) Representative: Plougmann Vingtoft a/s
(86) International application number: PCT/JP2023/040438
(87) International publication number: WO 2024/101428

(57) **Abstract**

The present invention relates to a biosignal measurement unit and a condition analysis system. When in a side view from a first earmuff (42R) of a biosignal measurement unit (14), a straight line parallel to a downwardly extending direction of a headband (40) and passing through a centroid (O1) of a plane region formed by the first earmuff (42R) is defined as a first reference line (LV), and a straight line orthogonal to the first reference line (LV) and passing through the centroid (O1) is defined a second reference line (LH), a first position (P3) is located at a position forward of the first reference line (LV) and downward of the second reference line (LH).

## Description

### Technical Field

The present invention relates to a biosignal measurement unit and a condition analysis system.

### Background Art

Conventionally, in various fields including medical, health, nursing care, and sports fields, there is a known technology that detects movements or conditions of a person to be measured by various sensors, and then analyzes various conditions of the person to be measured using the detection data obtained. As an example of such technology, a brain machine interface is known that analyzes electroencephalogram signals to identify a unique frequency indicating motor intention, a cognitive state, or a fatigue state of a person to be measured, and that operates a machine according to the signal strength of the unique frequency contained in the electroencephalogram signals.

An example of a fluctuation of brain waves that occurs at the unique frequency and that has a high correlation with motor intention is sensorimotor rhythm event-related desynchronization (hereinafter referred to as "SMR-ERD"). This SMR-ERD is estimated based on, for example, a signal showing the difference between a biosignal measured from an electrode part disposed at the top of the head and a biosignal measured from an electrode part disposed on the left motor cortex (or the right motor cortex) (hereinafter also referred to as "SMR-ERD signal").

Patent Document 1 discloses an electroencephalograph in which an earth electrode is disposed at a position rearward of the ear when the electroencephalograph is mounted on the head. When the earth electrode is provided at a place on the head that is less likely to be affected by biopotentials derived from brain activity as described above, it is possible to obtain a stable earth potential.

### Prior Art Document

### Patent Document

Patent Document 1: Japanese Patent Application Publication No. 2019-076712

### Non-Patent Document

Non-Patent Document 1: (1996) Human body dimensions data for ergonomic design edited by National Institute of Bioscience and Human-Technology, Japan Publication Service, ISBN4-88922-093-3 C3040 P4635E

Non-Patent Document 2: Du, et.al. "Head-and-Face Anthropometric Survey of Chinese Workers", Annual Occupational Hygiene Society, Vol. 52, No. 8, pp. 773-782, 2008, doi: 10.1093/annhyg/men056

Non-Patent Document 3: Gordon et al., "Anthropometric Survey of U.S. Army Personnel: Summary Statistics", Interim Report for 1988, UNITED STATES ARMY NATICKRESEARCH DEVELOPMENT AND ENGINEERING CENTER, Accession Number: ADA209600

### Disclosure of the Invention

### Problems to be Solved by the Invention

However, although the electroencephalograph disclosed in Patent Document 1 can perform measurements or analyses related to brain activity, the electroencephalograph cannot perform measurements or analyses related to muscle activity that occurs in the vicinity of the head, including the masseter muscle or the sternocleidomastoid muscle.

The present invention has been made in view of such a problem, and it is an object of the present invention to provide a biosignal measurement unit and a condition analysis system that can perform, with a simpler device configuration, various measurements or analyses by taking into account muscle activity that occurs in the vicinity of the head.

### Means for Solving the Problems

A biosignal measurement unit according to the present invention includes: a headband having an inverted U-shape, and being mountable on a head of a person to be measured; a first earmuff provided at one end part of the headband, and configured to cover one ear of the head with the headband mounted; a second earmuff provided at the other end part of the headband, and configured to cover the other ear of the head with the headband mounted; a first protruding member extending from a first position located at an outer edge part of the first earmuff; and a first muff-side electrode part provided to the first protruding member at a position close to a distal end of the first protruding member, the first muff-side electrode part being configured to measure a biosignal in the person to be measured, wherein, when in a side view from the first earmuff, a straight line parallel to a downwardly extending direction of the headband and passing through a centroid of a plane region formed by the first earmuff is defined as a first reference line, and a straight line orthogonal to the first reference line and passing through the centroid is defined as a second reference line, the first position is located forward of the first reference line and downward of the second reference line.

At least a distal end part of the first protruding member may be provided to extend toward a forward and inward direction in an inclined manner relative to the first position.

The biosignal measurement unit may further include a band-side electrode part attached to the headband, and configured to measure a biosignal in the head, and a difference between a first biosignal acquired from the band-side electrode part and a second biosignal acquired from the first muff-side electrode part may be amplified and outputted.

The biosignal measurement unit may further include: a second protruding member extending from a second position located at an outer edge part of the second earmuff; and a second muff-side electrode part provided to the second protruding member at a position close to a distal end of the second protruding member, the second muff-side electrode part being configured to measure a biosignal in the person to be measured, wherein when in a side view from the second earmuff, a straight line parallel to the downwardly extending direction of the headband and passing through a centroid of a plane region formed by the second earmuff is defined as a third reference line, and a straight line orthogonal to the third reference line and passing through the centroid is defined as a fourth reference line, the second position may be located at a position rearward of the third reference line and downward of the fourth reference line.

A condition analysis system according to the present invention includes: any of the above-mentioned biosignal measurement units; and a condition analysis device configured to perform analysis processing on the biosignal, which is measured by the biosignal measurement unit, to analyze motor intention, a cognitive state, or a fatigue state of the person to be measured.

### Effects of the Invention

According to the present invention, it is possible to perform, with a simpler device configuration, various measurements or analyses by taking into account muscle activity that occurs in the vicinity the head.

### Brief Description of the Drawings

FIG. 1 is an overall configuration diagram of a condition analysis system according to one embodiment of the present invention.
FIG. 2 is a front view of a headset shown in FIG. 1.
FIG. 3 is a plan view of the headset shown in FIG. 1.
FIG. 4 is a bottom view of the headset shown in FIG. 1.
FIG. 5 is a right side view of the headset shown in FIG. 1.
FIG. 6 is a schematic view showing the positional relationship between three electrode parts.
FIG. 7 is a diagram schematically showing the positional relationship between the three electrode parts and target regions.
FIG. 8 is a graph showing an example of variation over time of electroencephalogram signals.
FIG. 9 is a diagram schematically showing an activation region of SMR-ERD signals.
FIG. 10 is a diagram schematically showing a high correlation region of the SMR-ERD signals.
FIG. 11 is a diagram showing the basis for deciding a target region.
FIG. 12 is a diagram showing a person to be measured on which the headset is mounted, as viewed from the right side.
FIG. 13 is a diagram of the person to be measured on which the headset is mounted, as viewed from the left side.

### Mode for Carrying Out the Invention

Hereinafter, an embodiment of the present invention will be described with reference to attached drawings. To facilitate understanding of the description, identical constitutional elements in respective drawings are given the same reference symbols as far as possible, and a repeated description will be omitted.

### [Configuration of condition analysis system 10]

### <Overall configuration>

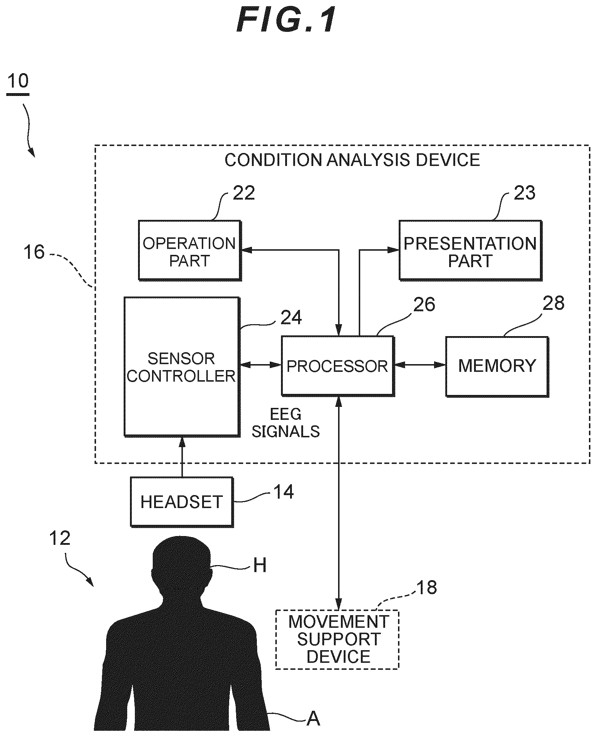

FIG. 1 is an overall configuration diagram of a condition analysis system 10 according to one embodiment of the present invention. This condition analysis system 10 serves as a brain machine interface system (hereinafter, BMI system) that analyzes electroencephalogram signals emitted from a person to be measured 12, and that supports movement of the person to be measured 12 based on the analysis result. To be more specific, the condition analysis system 10 is configured to include a headset 14 (corresponding to "biosignal measuring device"), a condition analysis device 16, and a movement support device 18.

The headset 14 is configured to be capable of measuring electric signals (that is, biosignals) correlating with the condition of the person to be measured 12 in a state of being mounted on a head H of the person to be measured 12. The headset 14 measures biosignals via a plurality of electrode parts, and outputs the obtained biosignals to the condition analysis device 16 by wireless communication or wired communication.

The condition analysis device 16 is a computer configured to be capable of analyzing the condition (for example, motor intention, a cognitive state, or a fatigue state) of the person to be measured 12 based on the biosignals measured by the headset 14. To be more specific, this condition analysis device 16 includes an operation part 22, a presentation part 23, a sensor controller 24, a processor 26, and a memory 28.

The operation part 22 is configured to allow a user, including the person to be measured 12 or a medical worker, to perform various operations. The operation part 22 is an input device including operation buttons and a microphone, or is an output device including a display panel and a speaker, for example.

The presentation part 23 is an output device that presents, to the person to be measured 12, information for requesting the person to be measured 12 to assume a rest state (hereinafter also referred to as request information) in response to commands from the processor 26. The presentation part 23 is constituted of, for example, a display panel, a lamp, a speaker, and the like. Examples of a presentation mode of the request information include guidance given by text or voice, turning on of the lamp, and outputting of various kinds of sound. A main body that presents the request information is not limited to the presentation part 23 of the condition analysis device 16, and may be a person different from the person to be measured 12 (an operator of the condition analysis device 16, for example).

The sensor controller 24 is a control circuit that performs various controls of the headset 14. The sensor controller 24 can perform various kinds of signal processing, such as sampling processing including synchronization of sensors, low-pass filter processing, and A/D conversion processing, for example. Consequently, the sensor controller 24 acquires electric signals (that is, biosignals) correlating with the condition of the person to be measured 12 at a predetermined sampling interval, and supplies these biosignals to the processor 26. To be more specific, the sampling interval may take any value within a range of from 0.5 to several hundred milliseconds.

The processor 26 centrally controls respective components constituting the condition analysis device 16. The processor 26 may be a general purpose processor including a CPU (central processing unit) or an MPU (micro-processing unit), or may be a dedicated processor including an FPGA (field programmable gate array) or a GPU (graphics processing unit).

The memory 28 is a non-transitory storage medium including a ROM (read only memory) and a RAM (random access memory), and stores programs and data necessary for the processor 26 to control respective constitutional elements.

The movement support device 18 is, for example, an actuator that can be mounted on an object part (an arm A in the example of this drawing) of the person to be measured 12. Examples of the object part include various body parts that perform stretching or bending movement, such as hands, legs, fingers, knees, and elbows. This movement support device 18 may have any configuration, and may be excluded from the condition analysis system 10 when required.

### <Configuration of headset 14>

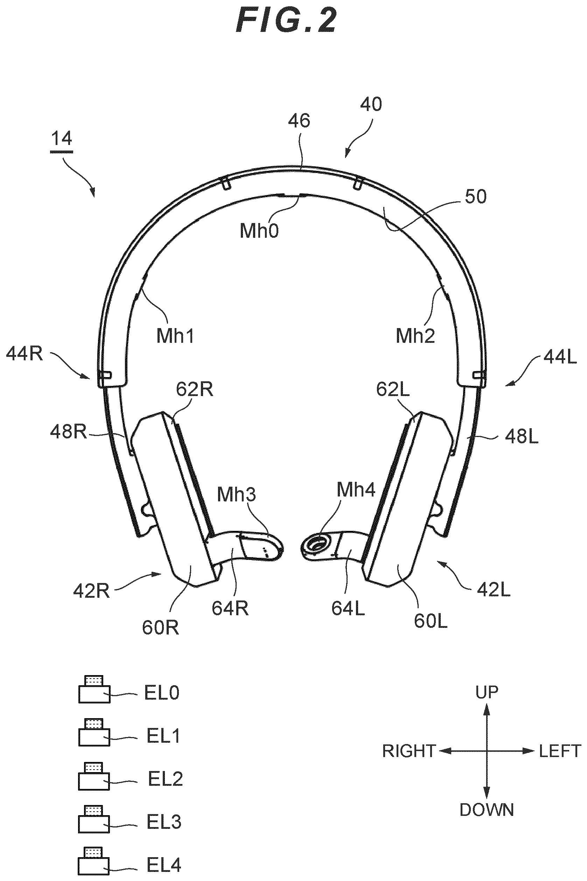

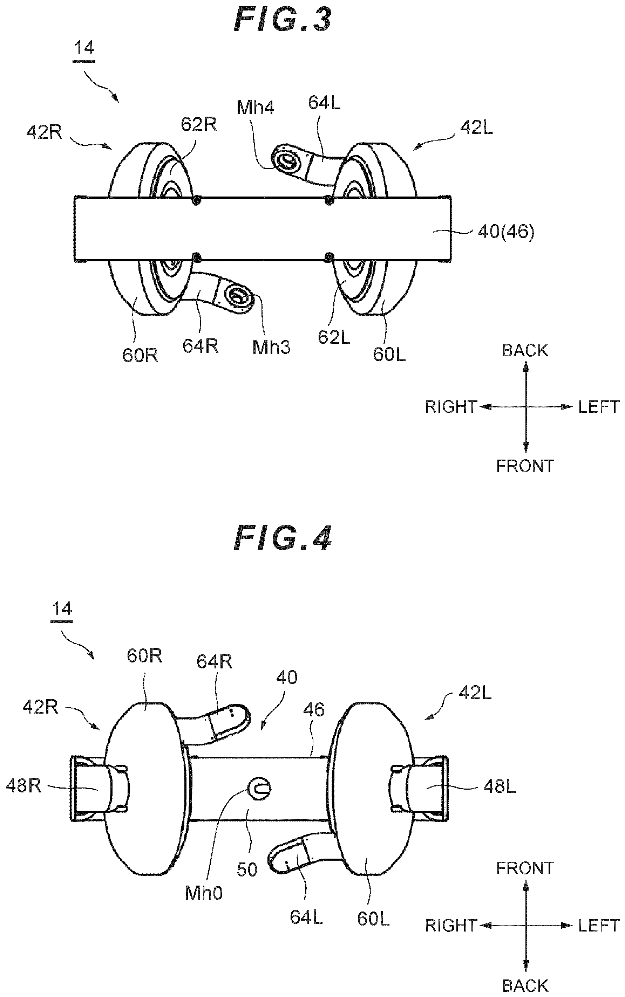

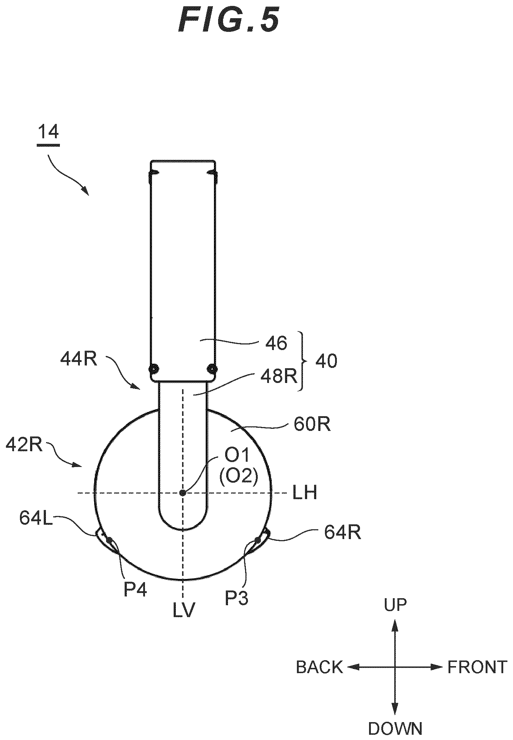

Subsequently, the configuration of the headset 14 will be described with reference to FIG. 2 to FIG. 6. FIG. 2 is a front view of the headset 14 shown in FIG. 1. FIG. 3 is a plan view of the headset 14 shown in FIG. 1. FIG. 4 is a bottom view of the headset 14 shown in FIG. 1. FIG. 5 is a right side view of the headset 14 shown in FIG. 1.

Hereinafter, the front-rear direction, the left-right direction, and the up-down direction are defined relative to the direction of the head H of the person to be measured 12 with the headset 14 correctly mounted on the head H. Although FIG. 2 to FIG. 5 show a state in which five electrode parts EL0 to EL4 (FIG. 2) are removed, in practice, the headset 14 is used in a state in which the electrode parts EL0 to EL4 are attached.

As shown in FIG. 2 to FIG. 5, the headset 14 includes one headband 40, an earmuff 42R (corresponding to "first earmuff"), and an earmuff 42L (corresponding to "second earmuff"), the earmuff 42R being provided at the right end part of the headband 40, the earmuff 42L being provided at the left end part of the headband 40.

The headband 40 is a strip-shaped member having an inverted U-shape, and is configured to include an insulating material, such as a resin. The headband 40 is provided with a length adjusting mechanism 44R (corresponding to "first adjusting mechanism") and a length adjusting mechanism 44L (corresponding to "second adjusting mechanism"), the length adjusting mechanism 44R being provided for adjusting the length of the headband 40 on the right side, the length adjusting mechanism 44L being provided for adjusting the length of the headband 40 on the left side.

The length adjusting mechanism 44R is constituted of a band body 46 and a slide member 48R, the band body 46 having a substantially arc shape, the slide member 48R being provided in such a way as to be expandable and contractable by sliding from the right end part of the band body 46. The length adjusting mechanism 44L is constituted of the above-mentioned band body 46 and a slide member 48L which is provided in such a way as to be expandable and contractable by sliding from the left end part of the band body 46.

An inner side surface 50 of the band body 46 has three hole parts Mh0, Mh1, Mh2. The hole part Mh0 at the center is provided in such a way as to allow the electrode part EL0 (corresponding to "center electrode part") to be attached to and detached from the hole part Mh0. The hole part Mh1 on the right side is provided in such a way as to allow the electrode part EL1 (corresponding to "first electrode part") to be attached to and detached from the hole part Mh1. The hole part Mh2 on the left side is provided in such a way as to allow the electrode part EL2 (corresponding to "second electrode part") to be attached to and detached from the hole part Mh2. The electrode parts EL0 to EL2 are configured to include metal electrodes used to acquire biosignals. Examples of the metal electrode include a silver-silver chloride electrode, a silver electrode, a gold electrode, and a platinum electrode. The electrode parts EL0 to EL2 may be constituted of electrodes of one kind, made of the same material and having the same thickness, or may be constituted of electrodes of two or more kinds, made of different materials or having different thicknesses.

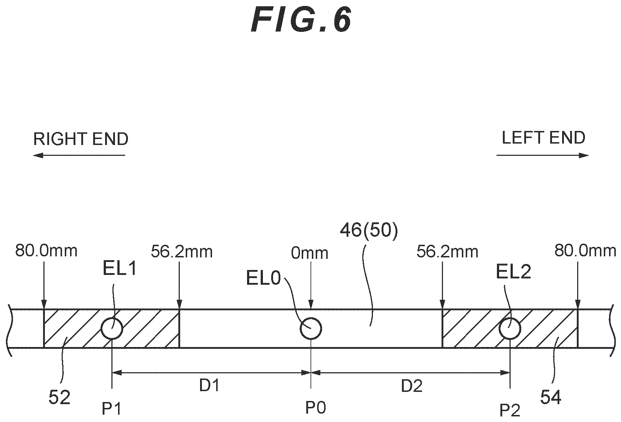

FIG. 6 is a diagram schematically showing the positional relationship between the three electrode parts EL0 to EL2. This drawing shows the band body 46 in a state in which the bent band body 46 is developed horizontally. The electrode part EL0 is disposed at a position P0 located at substantially the center of the band body 46 in the extending direction of the band body 46. The electrode part EL1 is disposed at a position P1 located on the right of the position P0. The electrode part EL2 is disposed on the band body 46 at a position P2 located on the left of the position P0. Assuming a distance between the positions P0, P1 of two points as "D1" (unit: mm), and a distance between the positions P0, P2 of two points as "D2" (unit: mm), the relationship of D1 = D2 is satisfied. Alternatively, the dimensional relationship of either one of D1> D2 or D1 < D2 may be satisfied.

The position P1 is within an allowable region 52 which is set in relation to the position P0. This allowable region 52 is a rectangular region to the right of the position P0 and lies within a range of from 56.2 to 80.0 mm from the position P0. That is, the distance D1 satisfies the dimensional relationship of 56.2 ≤ D1 ≤ 80.0.

The position P2 is within an allowable region 54 which is set in relation to the position P0. This allowable region 54 is a rectangular region to the left of the position P0 and lies within a range of from 56.2 to 80.0 mm from the position P0. That is, the distance D2 satisfies the dimensional relationship of 56.2 ≤ D2 ≤ 80.0.

Returning to FIG. 2 to FIG. 5, the earmuff 42R on the right side is configured to include a casing 60R, an earcup 62R, and a protruding member 64R.

The casing 60R has a bottomed cylindrical shape with an upper opening, and is configured to include an insulating material, such as a resin. The casing 60R houses a sensor substrate not shown in the drawing. Various electronic components are mounted on this sensor substrate, the various electronic components acquiring and outputting biosignals in the person to be measured 12 to the condition analysis device 16 (FIG. 1). The bottom part of the casing 60R is swingably connected to the right end part of the headband 40 (that is, the distal end part of the slide member 48R).

The earcup 62R has a disk shape, and is configured to include an insulating material, such as a resin. The earcup 62R is provided in such a way as to cover the opening part of the casing 60R. The main surface of the earcup 62R has an annular recessed and projecting part for performing positioning of the left ear of the person to be measured 12.

The protruding member 64R has a shape bent in a C shape, and is configured to include an insulating material, such as a resin. The protruding member 64R is provided to extend in the inward and forward direction from the outer edge part (a position P3 in FIG. 5) of the casing 60R. The protruding member 64R has one hole part Mh3 at a position close to the distal end thereof. This hole part Mh3 is provided in such a way as to allow the electrode part EL3 (corresponding to "first muff-side electrode part") to be attached to and detached from the hole part Mh3.

In contrast, the earmuff 42L on the left side is, in the same manner as the earmuff 42R on the right side, configured to include a casing 60L, an earcup 62L, and a protruding member 64L.

The casing 60L has a bottomed cylindrical shape with an upper opening, and is configured to include an insulating material, such as a resin. The casing 60L houses a sensor substrate not shown in the drawing. Various electronic components are mounted on this sensor substrate, the various electronic components acquiring and outputting biosignals in the person to be measured 12 to the condition analysis device 16 (FIG. 1). The bottom part of the casing 60L is swingably connected to the left end part of the headband 40 (that is, the distal end part of the slide member 48L).

The earcup 62L has a disk shape, and is configured to include an insulating material, such as a resin. The earcup 62L is provided in such a way as to cover the opening part of the casing 60L. The main surface of the earcup 62L has an annular recessed and projecting part for performing positioning of the left ear of the person to be measured 12.

The protruding member 64L has a shape bent in a C shape, and is configured to include an insulating material, such as a resin. The protruding member 64L is provided to extend in the inward and rearward direction from the outer edge part (a position P4 in FIG. 5) of the casing 60L. The protruding member 64L has one hole part Mh4 at a position close to the distal end thereof. This hole part Mh4 is provided in such a way as to allow the electrode part EL4 (corresponding to "second muff-side electrode part") to be attached to and detached from the hole part Mh4.

As shown in FIG. 5, the centroid position of the plane region formed by the earmuff 42R (to be more specific, the casing 60R) when the headset 14 is viewed from the right side is defined as "O1". A straight line that is parallel to the downwardly extending direction of the headband 40 (hereinafter, first direction) and that passes through the centroid position O1 is defined as "vertical reference line LV" (corresponding to "first reference line"). A straight line that extends in the direction (hereinafter, second direction) orthogonal to the first direction and that passes through the centroid position O1 is defined as "horizontal reference line LH" (corresponding to "second reference line"). In this case, the position P3 is located at a position forward of the vertical reference line LV and downward of the horizontal reference line LH.

In the same manner, the centroid position of the plane region formed by the earmuff 42L (to be more specific, the casing 60L) when the headset 14 is viewed from the left side is defined as "O2". A straight line that is parallel to the first direction, being the downwardly extending direction of the headband 40, and that passes through the centroid position O1 is defined as "vertical reference line LV" (corresponding to "third reference line"). A straight line that extends in the second direction orthogonal to the first direction and that passes through the centroid position O2 is defined as "horizontal reference line LH" (corresponding to "fourth reference line"). In this case, the position P4 is located at a position rearward of the vertical reference line LV and downward of the horizontal reference line LH.

### [First manner of operation and advantageous effects]

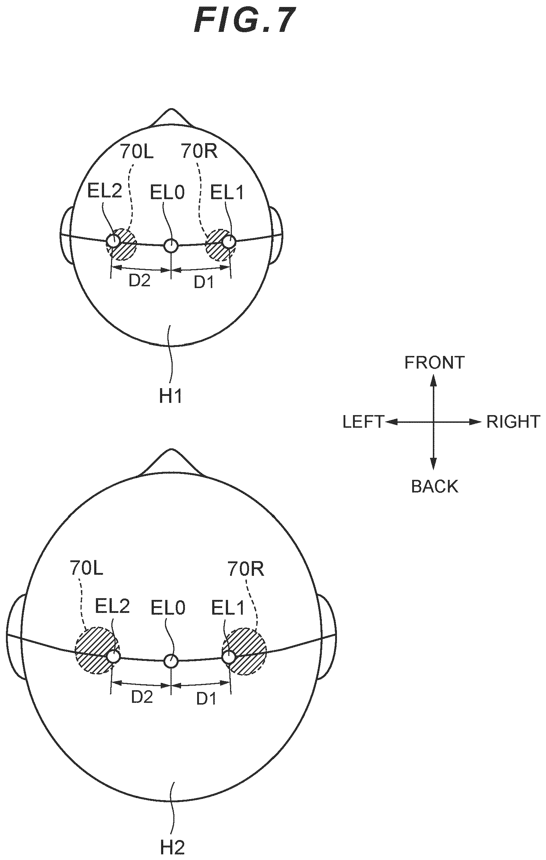

The condition analysis system 10 according to the present embodiment has the above-mentioned configuration. Next, the first manner of operation and advantageous effects brought about by the headset 14, which constitutes a portion of the condition analysis system 10, will be described with reference to FIG. 7 to FIG. 11 in addition to the above-mentioned FIG. 1 to FIG. 6.

### <Manner of operation>

Assume a case in which the condition analysis system 10 is used to identify an individual SMR-ERD frequency (that is, ISF) specific to the person to be measured 12. In the present embodiment, "SMR-ERD frequency" refers to a frequency at which event-related desynchronization (ERD), being movement-related response, is strong in the alpha band of 8 to 13 Hz of scalp electroencephalogram measured near the motor cortex. It is known that the level of the SMR-ERD frequency differs depending on the person, and fluctuates within a range of from 8 to 13Hz. Therefore, to clearly indicate a frequency unique to an individual, there may be cases in which an SMR-ERD frequency is referred to as "individual SMR-ERD frequency" (that is, ISF).

The headset 14 shown in FIG. 2 to FIG. 6 is used to obtain electroencephalogram signals that contain many spectral components at the SMR-ERD frequency. Prior to the measurement of electroencephalogram signals, the person to be measured 12 mounts the headset 14 on the head H of the person to be measured 12. To be more specific, the person to be measured 12 grips the headset 14 with both hands to fix one earmuff 42R to the right ear, and to fix the other earmuff 42L to the left ear. Thereafter, the person to be measured 12 adjusts the length of the headband 40 using the length adjusting mechanisms 44R, 44L of the headband 40 such that the inner side surface 50 of the headband 40 fits the surface of the head H. When this length adjustment is performed, positioning of the three electrode parts EL0, EL1, EL2 is performed simultaneously.

FIG. 7 is a diagram schematically showing the positional relationship between the three electrode parts EL0 to EL2 and target regions 70R, 70L. In the present embodiment, assume a case in which the same headset 14 is mounted on each of two persons to be measured 12 having different sizes of the head H. For the sake of convenience of the description, heads H1, H2 of the persons to be measured 12 are shown by elliptical shapes. When the above-mentioned positioning of the headset 14 is performed, the electrode part EL0 is disposed at the top of the head irrespective of the size of the head H1, H2.

However, the position of a target region 70R for the right motor cortex (or a target region 70L for the left motor cortex) varies depending on the size of the head H1, H2. These target regions 70R, 70L indicate regions that allow measurement of electroencephalogram signals (that is, SMR-ERD signals) containing spectral components at the SMR-ERD frequency with even higher accuracy. When the three electrode parts EL0 to EL2 satisfy the positional relationship shown in FIG. 6, it is possible to simultaneously measure electroencephalogram signals within the target regions 70R, 70L using the same headset 14 irrespective of "variation" in the size of the head H1, H2.

### <Basis for target regions 70R, 70L>

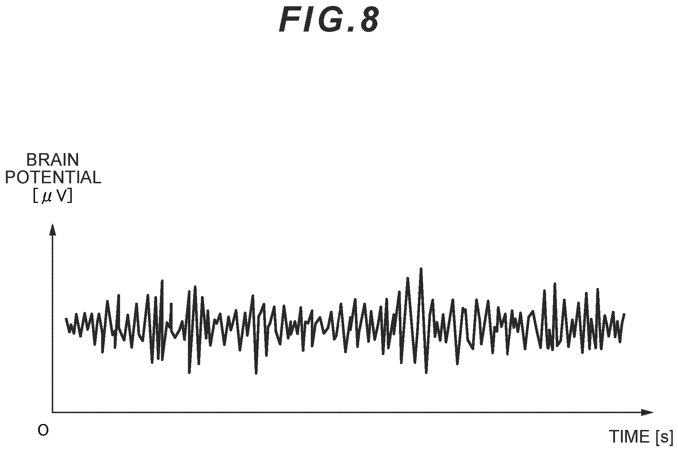

Subsequently, the basis for deciding positions and sizes of the target regions 70R, 70L shown in FIG. 7 will be described with reference to FIG. 8 to FIG. 11. To individually decide the target regions 70R, 70L, the inventors of the present invention acquired electroencephalogram data from 82 subjects, and analyzed the electroencephalogram data. These electroencephalogram data were acquired by using an electroencephalograph measuring device that can measure high-density electroencephalograms in 129 channels across the entire head. By performing such an analysis, it was possible to obtain detailed distribution of activation regions of the SMR-ERD signals.

FIG. 8 is a graph showing variation over time of electroencephalogram signals when the subject is at rest. The horizontal axis of the graph shows time (unit: s), and the vertical axis of the graph shows brain potential (unit: µV). As can be understood from this graph, the electroencephalogram signal has complex waveforms that finely fluctuate upward and downward about a reference value.

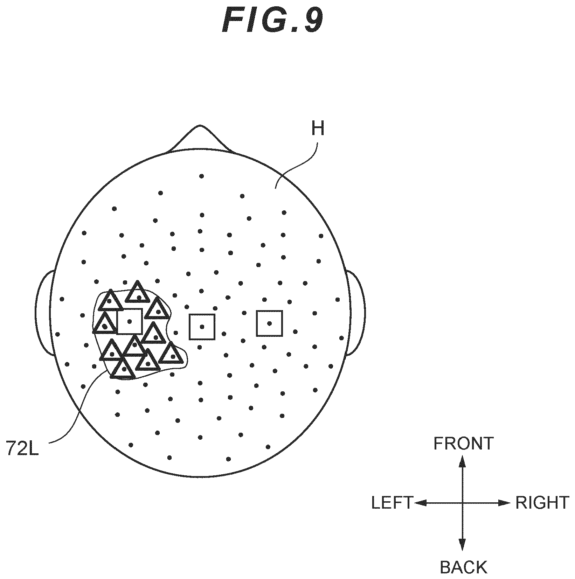

FIG. 9 is a diagram schematically showing an activation region 72L of SMR-ERD signals. Each dot within a circular region in this drawing indicates a measurement position of electroencephalogram signals on the head H. Three dots surrounded by squares indicate, from left to right, a C3 position, a Cz position, and a C4 position in the international 10-20 system. Each dot surrounded by a triangle corresponds to a measurement position determined to have activation in relation to the electroencephalogram signal acquired at the C3 position. That is, a closed curve enveloping all triangles defines a region that approximates the amount of activation at the C3 position (hereinafter, referred to as "activation region 72L").

For example, in a time period in which the ERD value at the C3 position is a positive value, (ERD value - standard deviation) at the C3 position is used as a threshold and, when the ERD value at each measurement position exceeds the threshold, it is determined that the measurement position belongs to the activation region 72L. In the present embodiment, the number of samples of the person to be measured 12 is 82 (N = 82), and the ERD value is obtained such that a median is obtained from time series data of each person to be measured 12, and the ERD value is calculated from the mean value of these medians (N = 82).

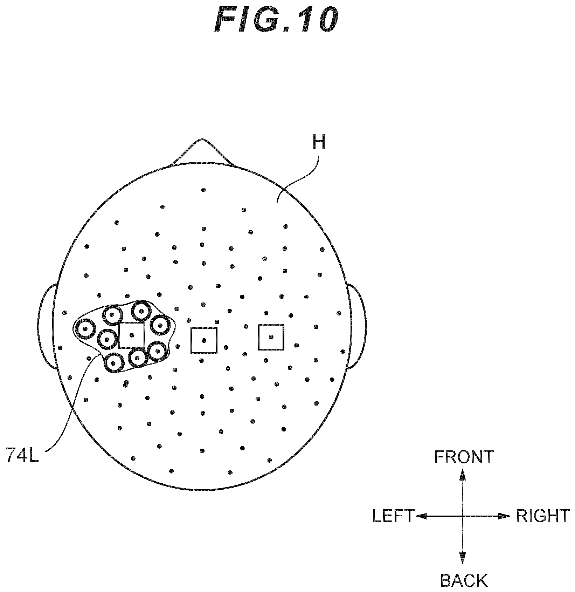

FIG. 10 is a diagram schematically showing a high correlation region 74L of the SMR-ERD signals. Each dot within the circular region in this drawing indicates the measurement position of electroencephalogram signals on the head H. Three dots surrounded by squares indicate, from left to right, the C3 position, the Cz position, and the C4 position in the international 10-20 system. Each dot surrounded by a circle corresponds to a measurement position determined to have a high time correlation in relation to an electroencephalogram signal acquired at the C3 position. That is, a closed curve enveloping all circles defines a region that approximates time series signals at the C3 position (hereinafter, referred to as "high correlation region 74L").

For example, in a time period in which the ERD value at the C3 position is a positive value, a correlation coefficient between time series data of the ERD value at the C3 position and time series data of the ERD value at each measurement position is obtained and, when the correlation coefficient exceeds a predetermined threshold (for example, 0.55), it is determined that the measurement position belongs to the high correlation region 74L. In the present embodiment, the number of samples of the person to be measured 12 is 82 (N = 82), and the correlation coefficient is calculated from the mean value of respective values obtained for the respective persons to be measured 12 (N = 82).

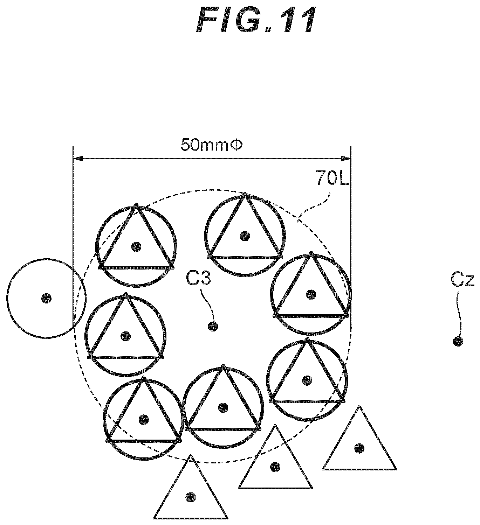

FIG. 11 is a diagram showing the basis for deciding the target region 70L. This drawing corresponds to a partial enlarged view of the C3 position in FIG. 9 and FIG. 10. Each dot surrounded by a triangle corresponds to the measurement position belonging to the activation region 72L, and each dot surrounded by a circle corresponds to the measurement position belonging to the high correlation region 74L. That is, each dot surrounded by both a triangle and a circle belongs to an overlapping region (that is, the target region 70L) between the activation region 72L and the high correlation region 74L. As can be understood from this drawing, this target region 70L has a circular shape about the C3 position with a diameter of 50 mm (a radius of 25 mm). As described above, the inventors of the present invention have found that, by arranging the electrode part EL2 within the target region 70L having a circular shape with a diameter of 50 mm, it is possible to acquire SMR-ERD signals.

Although the description has been made for the relationship between the target region 70L on the left side and the C3 position in the example shown in FIG. 9 to FIG. 11, the same applies for the relationship between the target region 70R on the right side and the C4 position. The reason for this is that bilateral symmetry of a brain function is basically observed in the SMR-ERD frequency. The inventors of the present invention have found that, in the same manner as the above description, by arranging the electrode part EL1 within the target region 70R having a circular shape with a diameter of 50 mm, it is possible to measure SMR-ERD signals.

### <Basis for arrangement of electrode parts EL0 to EL2>

Next, the basis for deciding the arrangement of the electrode parts EL0 to EL2 that is applicable to various heads H will be described. In the above-mentioned Non-Patent Document 1, dimensional data for "bitragion coronal arc" of Japanese persons were obtained by performing measurements on 250 men and 250 women between 18 and 88 years old, that is, 500 people in total. In the above-mentioned Non-Patent Document 2, dimensional data for "bitragion coronal arc" of Chinese persons were obtained by performing measurements on 2026 men and 974 women between 18 and 66 years old, that is, 3000 people in total. In the above-mentioned Non-Patent Document 3, dimensional data for "bitragion coronal arc" of American persons were obtained by performing measurements on 1774 men and 2208 women between 18 and 88 years old, that is, 3982 people in total. A bitragion coronal arc is a length from one tragion to the other tragion via the vertex, and is measured in a direction perpendicular to the orbito-meatal plane. This length is measured using a tape measure in a state in which a person to be measured sits on a chair.

As described in the above-mentioned Non-Patent Document 1, in the human body dimensions DB of Japanese persons, the minimum value of the bitragion coronal arc is 316 mm, and the maximum value of the bitragion coronal arc is 405 mm. When the electrode parts EL1, EL2 are located within ranges (that is, the target regions 70R, 70L) that allow measurements of SMR-ERD signals for both the maximum value and the minimum value, it is possible to perform measurements of SMR-ERD signals even for intermediate values between the maximum value and the minimum value.

At this time, the area being focused on is an area about C3 (or C4) with a radius of 25 mm. Prior to calculation of this area, a distance between Cz and C3 in the international 10-20 system is obtained. According to the definition of the international 10-20 system, the distance between Cz and C3 corresponds to 20% of the entire bitragion coronal arc. That is, regarding the distance between Cz and C3, the minimum value is 316 × 0.2 = 63.2 mm, and the maximum value is 405 mm × 0.2 = 81.0 mm.

As described above, the radius of the target region 70R, 70L is 25 mm and hence, in the case of a head H having the minimum size, it is sufficient that the measurement position fall within a range of from 63.2 mm ± 25 mm = 38.2 to 88.2 mm. In contrast, in the case of a head H having the maximum size, it is sufficient that the measurement position fall within a range of from 81.0 mm ± 25 mm = 56.0 to 106.0 mm. With such setting, the measurement position is provided within a product set that covers the above-mentioned two ranges, that is, within a range of from 56.0 to 88.2 mm, and hence, it is possible to perform measurements of SMR-ERD signals for most Japanese persons.

Although the above-mentioned example shows a method for deciding a measurement range suitable for the case in which Japanese persons are taken as a population, it is possible to calculate a measurement range suitable for other populations (for example, Chinese persons, American persons) by a similar method.

As described in the above-mentioned Non-Patent Document 2, in the human body dimensions DB of Chinese persons, the minimum value of the bitragion coronal arc is 275 mm, and the maximum value of the bitragion coronal arc is 406 mm. Regarding a distance between Cz and C3, the minimum value is 275 × 0.2 = 55.0 mm, and the maximum value is 406 mm × 0.2 = 81.2 mm. In the case of a head H having the minimum size, it is sufficient that the measurement position fall within a range of from 55.0 mm ± 25 mm = 30.0 to 80.0 mm. In the case of a head H having the maximum size, it is sufficient that the measurement position fall within a range of from 81.2 mm ± 25 mm = 56.2 to 106.2 mm. With such setting, the measurement position is provided within a product set that covers the above-mentioned two ranges, that is, within a range of from 56.2 to 80.0 mm, and hence, it is possible to perform measurements of SMR-ERD signals for most Chinese persons.

As described in the above-mentioned Non-Patent Document 3, in the human body dimensions DB of American persons, the minimum value of the bitragion coronal arc is 298 mm, and the maximum value of the bitragion coronal arc is 392 mm. Regarding a distance between Cz and C3, the minimum value is 298 × 0.2 = 59.6 mm, and the maximum value is 392 mm × 0.2 = 78.4 mm. In the case of a head H having the minimum size, it is sufficient that the measurement position fall within a range of from 59.6 mm ± 25 mm = 34.6 to 84.6 mm. In the case of a head H having the maximum size, it is sufficient that the measurement position fall within a range of from 78.4 mm ± 25 mm = 53.4 to 103.4 mm. With such setting, the measurement position is provided within a product set that covers the above-mentioned two ranges, that is, within a range of from 53.4 to 84.6 mm, and hence, it is possible to perform measurements of SMR-ERD signals for most American persons.

With the above considerations, by providing the measurement position within a product set that covers all of three ranges of [1] the range of from 56.0 to 88.2 mm (for Japanese persons), [2] the range of from 56.2 to 80.0 mm (for Chinese persons), and [3] the range of from 53.4 to 84.6 mm (for American persons), that is, within a range of from 56.2 to 80.0 mm (the allowable regions 52, 54 in FIG. 6), it is possible to perform measurements of SMR-ERD signals for a large number of adults.

### <Advantageous effect>

As described above, the condition analysis system 10 according to the present embodiment includes a biosignal measurement unit (the headset 14 in the present embodiment) configured to measure biosignals in the person to be measured 12, and the condition analysis device 16 that performs analysis processing on the biosignals measured by the headset 14 to analyze motor intention, a cognitive state, or a fatigue state of the person to be measured 12.

The headset 14 includes the headband 40 and the plurality of electrode parts, the headband 40 having an inverted U-shape, and being mountable on the head H of the person to be measured 12, the plurality of electrode parts being attached to the headband 40 to measure biosignals in the head H. The plurality of electrode parts includes the center electrode part (the electrode part EL0 in the present embodiment) disposed at the position P0, being the center of the headband 40 in the extending direction of the headband 40, the first electrode part (the electrode part EL1 in the present embodiment) disposed at the position P1 separated from the position P0 by a predetermined distance D1 toward one end part of the headband 40, and the second electrode part (the electrode part EL2 in the present embodiment) disposed at the position P2 separated from the position P0 by a predetermined distance D2 toward the other end part of the headband 40. The predetermined distances D1, D2 are values selected from 56.2 to 80.0 mm.

As described above, the electrode parts EL0 to EL2 are disposed to have a positional relationship in which variation in the size of the head H of the person to be measured 12 is taken into account and hence, by performing positioning of disposing the electrode part EL0 at the top of the head, it is possible to dispose the remaining electrode parts EL1, EL2 within the desired target regions 70R, 70L simultaneously. Consequently, it is possible to suppress a reduction in accuracy of measurement of SMR-ERD signals caused by variations in the size of the head H, with a simpler device configuration, and without providing a mechanism that adjusts the positions of the electrode parts EL0 to EL2.

The headset 14 may further include the first earmuff (the earmuff 42R in the present embodiment) and the second earmuff (the earmuff 42L in the present embodiment), the first earmuff being provided to one end part of the headband 40, and being configured to cover one ear of the head H with the headband 40 mounted, the second earmuff being provided to the other end part of the headband 40, and being configured to cover the other ear of the head H with the headband 40 mounted. By covering both ears by a pair of earmuffs 42R, 42L, positioning of the headband 40 at least in the front-rear direction is performed.

The headband 40 may be configured to include the length adjusting mechanism 44R and the length adjusting mechanism 44L, the length adjusting mechanism 44R adjusting the length from the electrode part EL1 to the earmuff 42R, the length adjusting mechanism 44L adjusting the length from the electrode part EL2 to the earmuff 42L. When both ears are covered by the pair of earmuffs 42R, 42L and, thereafter, the length of the headband 40 is adjusted by using a pair of length adjusting mechanisms 44R, 44L, positioning of the headband 40 in the left-right direction is further performed.

The plurality of electrode parts may include electrode parts of two or more kinds having different thicknesses, and each of the electrode parts of two or more kinds may be configured to be attachable to and detachable from the headband 40. Consequently, it is possible to measure biosignals while the electrode part is replaced when necessary, and it is also possible to suppress a reduction in accuracy of measurement of SMR-ERD signals caused by a difference in thickness of the electrode part.

### [Second manner of operation and advantageous effects]

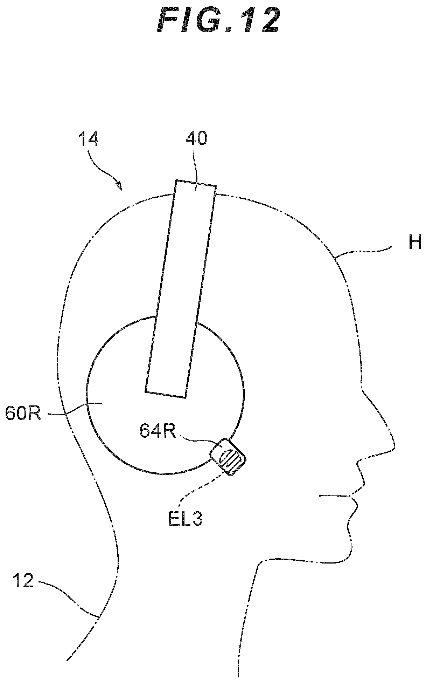

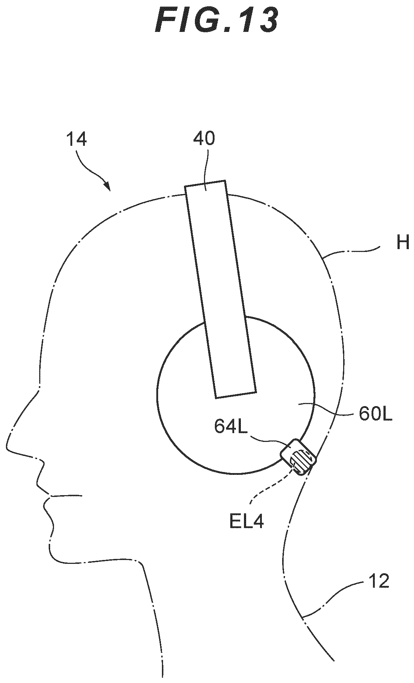

Next, the second manner of operation and advantageous effects brought about by the headset 14, which constitutes a portion of the condition analysis system 10, will be described with reference to FIG. 12 and FIG. 13 in addition to the above-mentioned FIG. 1 to FIG. 6.

### <Manner of operation>

Prior to the measurement of biosignals, the person to be measured 12 mounts the headset 14 on the head H of the person to be measured 12. To be more specific, the person to be measured 12 grips the headset 14 with both hands to fix one earmuff 42R to the right ear, and to fix the other earmuff 42L to the left ear.

FIG. 12 is a diagram of the person to be measured 12 on which the headset 14 is mounted, as viewed from the right side. The protruding member 64R provided to the earmuff 42R pushes a contact portion due to the action of the elastic force of the protruding member 64R, thus fixing the electrode part EL3 to the portion located forward and downward of the right ear. Consequently, it is possible to measure electric signals correlating with muscle activity that occurs in the vicinity of the head H including, for example, the masseter muscle or the sternocleidomastoid muscle.

FIG. 13 is a diagram of the person to be measured 12 on which the headset 14 is mounted, as viewed from the left side. The protruding member 64L provided to the earmuff 42L pushes a contact portion due to the action of the elastic force of the protruding member 64L, thus fixing the electrode part EL4 to a portion located rearward and downward of the left ear. By making use of the fact that a portion located rearward of the ear is less likely to be affected by biopotentials derived from brain activity, it is possible to acquire the ground potential (or the earth potential) from the electrode part EL4.

The condition analysis system 10 can perform various measurements or analyses in a state in which the headset 14 is mounted on the person to be measured 12.
[1] For example, the electrode part EL3 may be used as a reference electrode, and the electrode part EL4 may be used as a ground electrode. In this case, the headset 14 may output amplified signals of differences between electric signals from other electrode parts EL0 to EL2 and electric signals from the electrode part EL3. Such outputting allows, when muscle activity occurs, a potential change to be superimposed on other measurement channels (the electrode parts EL0 to EL2) in phase-inverted form through the above-mentioned differential amplification.
   Particularly, considering that brain activity significantly differs from muscle activity in the range of potential change, muscle activity can be used as "signals" more explicitly. In contrast, in the case in which muscle activity is considered to be "noise" against brain activity, by removing the zone in which muscle activity occurs from an analysis object, it is possible to increase accuracy in analyzing brain activity.
[2] In contrast, the electrode part EL3 may be used as a ground electrode, and the electrode part EL4 may be used as a reference electrode. In this case, the headset 14 may output amplified signals of differences between electric signals from other electrode parts EL0 to EL2 and electric signals from the electrode part EL4. Such outputting also allows a potential change caused by muscle activity that occurs in the vicinity of the head H to be superimposed on other measurement channels.
[3] The condition analysis device 16 may perform analysis processing related to muscle activity that occurs in the vicinity of the head H, in addition to analysis of brain activity. Examples of the analysis processing include (A) quantification of a stressed state of the person to be measured 12, (B) determination on whether the person to be measured 12 is in a rest state, and (C) specification of a target zone for electroencephalogram analysis.

### <Advantageous effect>

As described above, the biosignal measurement unit (the headset 14 in the present embodiment) according to the present embodiment includes the headband 40 having an inverted U-shape, and being mountable on the head H of the person to be measured 12, the first earmuff (the earmuff 42R in the present embodiment) provided at one end part of the headband 40, and configured to cover one ear of the head H with the headband 40 mounted, the second earmuff (the earmuff 42L in the present embodiment) provided to the other end part of the headband 40, and configured cover the other ear of the head H with the headband 40 mounted, the first protruding member (the protruding member 64R in the present embodiment) extending from the position P3 located at the outer edge part of the earmuff 42R, and the first muff-side electrode part (the electrode part EL3 in the present embodiment) provided to the protruding member 64R at a position close to the distal end of the protruding member 64R, the first muff-side electrode part being configured to measure biosignals in the person to be measured 12.

When in a side view from the earmuff 42R, a straight line parallel to a downwardly extending direction of the headband 40 and passing through the centroid position O1 in the plane region formed by the earmuff 42R is defined as the first reference line (the vertical reference line LV in the present embodiment), and a straight line orthogonal to the vertical reference line HV and passing through the centroid position O2 is defined as the second reference line (the horizontal reference line LH in the present embodiment), the position P3 is located at a position forward of the vertical reference line LV and downward of the horizontal reference line LH.

As described above, the position P3 located close to the proximal end of the protruding member 64R is located at the position forward of the vertical reference line LV and downward of the horizontal reference line LH and hence, by covering both ears by the pair of earmuffs 42R, 42L, the electrode part EL3 disposed at the position close to the distal end of the protruding member 64R is fixed to a portion located forward and downward of one ear of the head H. Consequently, it is possible to measure biosignals correlating with muscle activity that occurs in the vicinity of the head H, including the masseter muscle or the sternocleidomastoid muscle, and hence, it is possible to perform various measurements and various analyses in which the muscle activity is taken into consideration.

At least the distal end part of the protruding member 64R may be provided to extend toward the forward and inward direction in an inclined manner relative to the position P3. Consequently, the electrode part EL3 located at the position close to the distal end of the protruding member 64R easily comes into contact with the head H (to be more specific, a portion located forward and downward of the ear).

The headset 14 may further include the band-side electrode parts (the electrode parts EL0 to EL2 in the present embodiment) attached to the headband 40, and configured to measure biosignals in the head H, and may amplify and output differences between first biosignals acquired from the electrode parts EL0 to EL2 and second biosignals acquired from the electrode part EL3. Such outputting allows, when muscle activity occurs, a potential change to be superimposed on other electrode parts EL0 to EL2 in phase-inverted form through the differential amplification.

In the case in which the headset 14 further includes the second protruding member (the protruding member 64L in the present embodiment) extending from the position P4 located at the outer edge part of the earmuff 42L, and the second muff-side electrode part (the electrode part EL4 in the present embodiment) provided to the protruding member 64L at a position close to the distal end of the protruding member 64L to measure biosignals in the person to be measured 12, the position P4 may be located at a position rearward of the vertical reference line LV and downward of the horizontal reference line LH. By covering both ears by the pair of earmuffs 42R, 42L, the electrode part EL4 located at the position close to the distal end of the protruding member 64L is fixed to a portion located rearward and downward of the other ear of the head H. By making use of the fact that a portion located rearward of the ear is less likely to be affected by biopotentials derived from brain activity, it is possible to acquire the ground potential (or the earth potential) from the electrode part EL4.

### [Modification]

The present invention is not limited to the above-mentioned embodiment and, needless to say, the present invention can be freely modified without departing from the gist of the present invention. Alternatively, respective configurations may be suitably combined within an extent that no technical contradiction occurs.

In the above-mentioned embodiment, ideal arrangement of the electrode parts EL0 to EL2 is derived from the activation range of SMR-ERD signals obtained via an analysis using human body dimensional database described in Non-Patent Documents 1 to 3. If another signal is set as a target, it is assumed that the activation range varies depending on characteristics of the signal. In this case, based on the activation range obtained by an analysis, it is possible to set a measurement range applicable to a large number of people using a similar analysis method.

In the above-mentioned embodiment, the description has been made by taking, as an example, the case in which the protruding member 64R is fixed to the front part of the earmuff 42R, and the protruding member 64L is fixed to the rear part of the earmuff 42R. However, the configuration is not limited to such a configuration. For example, the protruding member 64R may be fixed to the rear part of the earmuff 42R, and the protruding member 64L may be fixed to the front part of the earmuff 42R. The configuration of at least one of the protruding members 64R, 64L may be omitted.

In the above-mentioned embodiment, the description has been made by taking, as an example, the case in which the protruding members 64R, 64L are fixed to the earmuffs 42R, 42L. However, instead of such a configuration, the protruding members 64R, 64L may be provided to the earmuffs 42R, 42L in such a way as to be movable in the circumferential direction. Consequently, it is possible to easily adjust the contact portions (that is, measurement positions) of the electrode parts EL3, EL4.

In the above-mentioned embodiment, the description has been made by taking, as an example, the case in which the condition analysis device 16 performs analysis of biosignals and control of the movement support device 18. However, the configuration of the system is not limited to such a configuration. For example, a configuration may be adopted in which the analysis unit and the control unit are provided as separate bodies, and necessary data can be exchanged between the analysis unit and the control unit by wired communication or wireless communication. In this case, a cloud server device or an on-premise server device may perform analysis processing. Particularly in the case in which the server device is a cloud server device, the server device may be formed of a group of computers that construct a distributed system.

### [Description of Reference Numerals]

- 10: condition analysis system
- 12: person to be measured
- 14: headset (biosignal measurement unit)
- 16: condition analysis device
- 40: headband
- 42R, 42L: earmuff
- EL0: electrode part (center electrode part)
- EL1: electrode part (first electrode part)
- EL2: electrode part (second electrode part)
- EL3: electrode part (muff-side first electrode part)
- EL4: electrode part (muff-side second electrode part)
- H, H1, H2: head

## Claims

1. A biosignal measurement unit comprising:
a headband having an inverted U-shape, and being mountable on a head of a person to be measured;
a first earmuff provided at one end part of the headband, and configured to cover one ear of the head with the headband mounted;
a second earmuff provided at the other end part of the headband, and configured to cover the other ear of the head with the headband mounted;
a first protruding member extending from a first position located at an outer edge part of the first earmuff; and
a first muff-side electrode part provided to the first protruding member at a position close to a distal end of the first protruding member, the first muff-side electrode part being configured to measure a biosignal in the person to be measured, wherein,
when in a side view from the first earmuff,
a straight line parallel to a downwardly extending direction of the headband and passing through a centroid of a plane region formed by the first earmuff is defined as a first reference line, and a straight line orthogonal to the first reference line and passing through the centroid is defined as a second reference line,
the first position is located forward of the first reference line and downward of the second reference line.

2. The biosignal measurement unit according to claim 1, wherein at least a distal end part of the first protruding member is provided to extend toward a forward and inward direction in an inclined manner relative to the first position.

3. The biosignal measurement unit according to claim 1, further comprising a band-side electrode part attached to the headband, and configured to measure a biosignal in the head, wherein
a difference between a first biosignal acquired from the band-side electrode part and a second biosignal acquired from the first muff-side electrode part is amplified and outputted.

4. The biosignal measurement unit according to claim 1, further comprising:
a second protruding member extending from a second position located at an outer edge part of the second earmuff; and
a second muff-side electrode part provided to the second protruding member at a position close to a distal end of the second protruding member, the second muff-side electrode part being configured to measure a biosignal in the person to be measured, wherein
when in a side view from the second earmuff,
a straight line parallel to the downwardly extending direction of the headband and passing through a centroid of a plane region formed by the second earmuff is defined as a third reference line, and a straight line orthogonal to the third reference line and passing through the centroid is defined as a fourth reference line,
the second position is located at a position rearward of the third reference line and downward of the fourth reference line.

5. A condition analysis system comprising:
the biosignal measurement unit according to any one of claims 1 to 4; and
a condition analysis device configured to perform analysis processing on the biosignal, which is measured by the biosignal measurement unit, to analyze motor intention, a cognitive state, or a fatigue state of the person to be measured.
